# EUROPÄISCHE PATENTSCHRIFT

(11) **EP 1 812 567 B1**
(45) Veröffentlichungstag und Bekanntmachung des Hinweises auf die Patenterteilung: **29.08.2012**
(21) Anmeldenummer: 05807871.8
(22) Anmeldetag: 10.11.2005
(51) Int. Cl.: C12N 11/14

(54) **VERFAHREN ZUR HERSTELLUNG BIOLOGISCH AKTIVEN MATERIALS, VERFAHRENSGEMÄSS HERGESTELLTES BIOLOGISCHES MATERIAL SOWIE MIT DIESEM VERSEHENE VORRICHTUNGEN**
METHOD FOR PRODUCING BIOLOGICALLY ACTIVE MATERIAL, BIOLOGICAL MATERIAL PRODUCED ACCORDING TO THIS METHOD, AND DEVICES PROVIDED THEREWITH
PROCÉDÉ POUR PRODUIRE UN MATÉRIAU BIOLOGIQUEMENT ACTIF, MATÉRIAU BIOLOGIQUE PRODUIT SELON CE PROCÉDÉ, AINSI QUE DISPOSITIFS MUNIS DE CE MATÉRIAU

(30) Priorität: 17.11.2004 DE 102004055547
(43) Veröffentlichungstag der Anmeldung: 01.08.2007
(73) Patentinhaber: EMW Filtertechnik GmbH, 65582 Diez (DE)
(72) Erfinder: BUCHER, Heinz, 65582 Diez (DE)
(74) Vertreter: Herden, Andreas F.
(86) Internationale Anmeldenummer: PCT/EP2005/012020
(87) Internationale Veröffentlichungsnummer: WO 2006/053671

(56) Entgegenhaltungen:
- US-A- 4 451 568
- US-A- 4 452 892
- PATENT ABSTRACTS OF JAPAN Bd. 012, Nr. 011 (C-468), 13. Januar 1988 (1988-01-13) & JP 62 166889 A (AGENCY OF IND SCIENCE & TECHNOL), 23. Juli 1987 (1987-07-23) -& DATABASE WPI Section Ch, Week 198735 Derwent Publications Ltd., London, GB; Class A96, AN 1987-245768 XP002365519 & JP 62 166889 A (AGENCY OF IND SCI & TECHNOLOGY) 23. Juli 1987 (1987-07-23)
- KLEIN J ET AL: "Immobilization of microbial cells by adsorption." JOURNAL OF BIOTECHNOLOGY. OCT 1990, Bd. 16, Nr. 1-2, Oktober 1990 (1990-10), Seiten 1-15, XP002365336 ISSN: 0168-1656
- PARK J K ET AL: "Microencapsulation of microbial cells" BIOTECHNOLOGY ADVANCES, ELSEVIER PUBLISHING, BARKING, GB, Bd. 18, Nr. 4, Juli 2000 (2000-07), Seiten 303-319, XP004213783 ISSN: 0734-9750
- COHEN Y: "Biofiltration--the treatment of fluids by microorganisms immobilized into the filter bedding material: a review." BIORESOURCE TECHNOLOGY. MAY 2001, Bd. 77, Nr. 3, Mai 2001 (2001-05), Seiten 257-274, XP002365337 ISSN: 0960-8524
- GROBOILLOT A ET AL: "IMMOBILIZATION OF CELLS FOR APPLICATION IN THE FOOD INDUSTRY" CRC CRITICAL REVIEWS IN BIOTECHNOLOGY, CRC PRESS, BOCA RATON, FL, US, Bd. 14, Nr. 2, 1994, Seiten 75-107, XP008058712 ISSN: 0738-8551

## Beschreibung

Die Erfindung betrifft allgemein ein Verfahren zur Herstellung biologisch aktiven Materials, das verfahrensgemäß hergestellte biologische Material sowie mit diesem versehene Vorrichtungen und betrifft im Besonderen ein Verfahren zur dauerhaften Festlegung von Mikroorganismen, insbesondere von katalytisch aktiven Mikroorganismen, an Trägermaterialien.

Der Einsatz von Mikroorganismen hat in den vergangenen Jahren große Bedeutung erlangt. So werden heutzutage Mikroorganismen wie Bakterien, Hefen, Pilze und/oder Algen in einer Vielzahl technischer Verfahren verwendet. Beispiele hierfür sind Einsatzbereiche in der Lebensmittelindustrie, wo diese bei der Herstellung von Bieren eingesetzt werden, in der Arzneimittelindustrie, in welcher diese bei der Herstellung von Hormonen und Antibiotika Verwendung finden, und in der Chemischen Industrie für die Herstellung von organischen Säuren und Alkoholen.

Darüber hinaus werden die vorstehend genannten Mikroorganismen derzeit in den unterschiedlichsten biotechnologischen Verfahren verwendet. Hierzu zählen die Abwasserreinigung, Bodensanierung und die Anwendung von Mikroorganismen in der Abluftreinigung mit Hilfe von Biofiltern und Biowäschern.

Ein bedeutender Vorteil bei der Verwendung von Mikroorganismen im Vergleich zur Verwendung von Enzymen oder Enzympräparaten ist es, dass hierdurch vermeidbar ist, aus den Zellen die Enzyme zu extrahieren, die Zellsubstanz abzutrennen und die Enzyme zu reinigen. Zudem sind Mikroorganismen besser in der Lage Folgereaktionen zu katalysieren und in situ die erforderlichen Cofaktoren zu regenerieren. Zudem stellen sie den Enzymen eine optimale Mikroumgebung zur Verfügung. Aus diesem Grund hat es in den vergangenen Jahren nicht an Versuchen gefehlt, Mikroorganismen zu stabilisieren bzw. immobilisieren um diese ohne nennenswerte Inaktivierung mit geringen Kosten zurück zu gewinnen und damit eine umfangreiche Verwendung in der Industrie zu ermöglichen.

Immobilisierte Mikroorganismen werden heutzutage als heterogene Katalysatoren verwendet und können nach Beendigung der Reaktion leicht aus dem Reaktionsgemisch entnommen werden. Dadurch können sie als Biokatalysatoren in Reaktoren mehrfach wiederverwendet werden.

Typische Beispiele für die genannten Verfahren sind in Acta. Chem. Scand., Bd 20, Seiten 2807-2810 (1966), Enzyme Microbial Tech, Band 1 Seite 95 (1979) beschrieben. Diese immobilisierten Mikroorganismen neigen jedoch beim Packen in einem Reaktor zur Deformation und bewirken einen hohen Druckverlust, was stark verringerte Fließgeschwindigkeiten und hohe Energieaufwendungen zur Folge hat.

Eine Stabilisierung der Gelmatrices in welche die Mikroorganismen eingebettet sind, wurde durch das Einbringen einer weiteren Komponente erreicht. Bei dieser handelt es sich beispielsweise um Silicate und Sand, wie in Biotechnol. Bioeng. Band 26, Seite 217-224 (1984) beschrieben wird. Zusätzliche Stabilisierung wird durch eine Quervernetzung der Matrix durch Reagenzien wie Glutaraldehyd erreicht.

Das Dokument JP 62 16689 zeigt ein Verfahren bei welchem eine PolymerLösung mit Mikroorganismen aufgebracht wird, welche auf der Oberfläche eines Substrats durch Vernetzung mit einem Metallsalz geliert.

Neben den Verfahren des Standes der Technik, wie vorher erwähnt, können Mikroorganismen ebenfalls in einer Gelmatrix aus sulfatiertem Polysaccharid (Carrageenan) eingeschlossen werden (Mikroeinkapselung), indem man eine wäßrige Mischung des Carrageenens und des Mikroorganismus abkühlt. Jedoch ist auch hier die erhaltene Gelmatrix zu weich und instabil, um bei Reaktionen mit wäßrigen Substraten verwendet werden zu können. So lockert diese in wäßrigem Millieu auf und ermöglicht den eingeschlossenen Mikroorganismen ein Heraussickern aus der Matrix. Eine Verwendung der vorstehend genannten fixierten Mikroorganismen in Anwendungsbereichen mit hohen Scherkräften ist daher nur schlecht möglich bzw. anwendungsabhängig teilweise sogar ausgeschlossen.

Weitere schwerwiegende Nachteile bei der Verwendung von immobilisierten Mikroorganismen sind die hohen Kosten für die Einbettungsmasse und der Verlust an katalytischer Aktivität und/oder die Schwierigkeit, die Mikroorganismen während des Immobilisierens nicht zu beschädigen. Ein Großteil der Mikroorganismen ist so instabil, daß diese beim Immobilisieren durch Umweltfaktoren, beispielsweise durch Temperatur, pH-Wert, Ionenstärke, Prozessdruck und dergleichen in deren enzymatischer Wirkung häufig sehr stark beeinträchtigt werden.

Es hat sich bisher als äußerst schwierig erwiesen, ein Verfahren zum Festlegen von Mikroorganismen an Trägermaterialien unter Vermeidung der vorstehend genannten Nachteile anzugeben oder zu verwirklichen.

Der Erfindung liegt insbesondere auch die Aufgabe zugrunde, die Verfügbarkeit von Mikroorganismen auf einem Substrat zu verbessern.

Die Aufgabe der Erfindung wird auf höchst überraschende weise bereits durch ein Verfahren mit den Merkmalen des Anspruchs 1 gelöst

Erfindungsgemäß ist vorgesehen, daß das Festlegen des Mikroorganismus an einem Substrat, einem Träger oder einer Trägereinrichtung unter Verwendung eines zumindest ein Acrylat enthaltenden Binde-, Klebe- oder Haltemittels erfolgt und/oder
daß das Festlegen des Mikroorganismus unter Verwendung eines zumindest den Mikroorganismus nicht vollständig umschließenden Binde-, Klebe- oder Haltemittels erfolgt.

Bevorzugte Ausführungsformen und Weiterentwicklungen der Erfindung sind Unteransprüchen zu entnehmen.

Die Erfindung beschreibt ein Verfahren zur Herstellung eines biologisch aktiven Materials, welches das dauerhafte Festlegen mindestens eines Mikroorganismus mit einem mindestens ein Acrylat enthaltenden Bindemittel an einem bereitgestellten Substrat umfaßt. In vorteilhafter Weise wird mit diesem Verfahren ein biologisch aktives Material hergestellt, in dem die Mikroorganismen dauerhaft an dem bereitgestellten Substrat gebunden oder an diesem befestigt sind und auch durch das Auftreten hoher Scherkräfte die unter dem Einfluß strömender Flüssigkeiten auf die Mikroorganismen einwirken, nicht von ihrem Träger gelöst werden.

Darüber hinaus stellt die Erfindung ein Verfahren bereit, mir dem es erstmals möglich ist, verschiedenartigste Substrate mit aktiven Mikroorganismen zu beschichten.

Die dauerhafte Festlegung des Mikroorganismus an der vielfältig gearteten Substratoberfläche wird durch Ionische Wechselwirkung, van der Waals-Wechselwirkung, physikalische Adsorption oder kovalentem Quervernetzen, ermöglicht.

Die Verfahren zur Herstellung des erfindungsgemäßen biologisch aktiven Materials sind vielfältig. So ist das Zustandekommen einer dauerhaften Festlegung zwischen Mikroorganismen und einer Substratoberfläche durch das Auftragen einer Mikroorganismen und zumindest ein Acrylat enthaltenden Bindemittelsuspension auf diese Oberfläche möglich.

Ebenfalls läßt sich auf ein bereitgestelltes Substrat eine Mikroorganismen enthaltende Suspension aufbringen und diese in einem darauffolgenden Arbeitsschritt mit Hilfe eines zumindest ein Acrylat enthaltenden Bindemittels dauerhaft auf dem bereitgestellten Substrat fixieren.

Ferner umfaßt die Erfindung die dauerhafte Festlegung von Mikroorganismen mit einem Substrat durch Aufbringen einer Mikroorganismen enthaltenden Suspension auf ein mit einem zumindest ein Acrylat enthaltenden Bindemittel überzogenem Substrat.

Das Aufbringen der Mikroorganismen und des Bindemittels kann unter Rücksichtnahme auf die Empfindlichkeit der verwendeten Mikroorganismen auf unterschiedliche Art und Weise geschehen. So kommt bei empfindlichen Mikroorganismen ein Aufbringen durch Tränken beziehungsweise Schlicker Suspension in Frage wohingegen robustere Species auf das entsprechende Substrat durch Besprühen aufgebracht werden können. Bei dem dauerhaften Festlegen von Mikroorganismen auf großporigen Substraten wie Beispielsweise Silikaten beziehungsweise Zeolithen kommt auch ein Füllen von Poren in Frage um einen vollständigen, stabilen Überzug der Substratoberfläche mit fixierten Mikroorganismen zu gewährleisten.

In Hinblick auf die Stabilität und Erhaltung der enzymatischen Aktivität der zu verbindenden Mikroorganismen sind möglichst milde Prozessparameter von größter Bedeutung um mögliche Denaturierungen der biologische wirksamen Enzyme zu verhindern. So hat es sich als vorteilhaft herausgestellt, die Temperatur während des Herstellungsprozesses zwischen etwa 10 bis etwa 100 °C, bevorzugt zwischen etwa 30 bis etwa 70 °C, besonders bevorzugt bei etwa 40 °C zu halten.

Bezüglich des pH-Wertes während der Herstellung sollten Vorkehrungen getroffen werden, daß pH-Werte von etwa 4,8 nicht unterschritten und pH-Werte von etwa 7 nicht überschritten werden.

In Hinblick auf die Herstellung des biologisch aktiven Materials hat es sich als vorteilhaft erwiesen, die Mikroorganismen mit einem Anteil an Bindemittel von 10 bis 90%, bevorzugt von 20 bis 60 %, besonders bevorzugt von 30 % vorzuvermischen.

Bei der Vorvermischung der Mikroorganismen mit dem Bindemittel haben sich in Vorversuchen Mischungszeiten von 1 h bis 24 h, bevorzugt von 8 h bis 20 h, besonders bevorzugt von 12 h herausgestellt.

Hinsichtlich der Belegungsdichte des mit Mikroorganismen zu Verbindenden Materials ermöglicht die Erfindung deren Variation abhängig von dem gewählten Einsatz des biologisch aktiven Materials. So haben sich in Vorversuchen Belegungsdichten zwischen etwa 1 und etwa 5.10⁶ Mikroorganismen/cm², bevorzugt zwischen etwa 1.10⁵ und etwa 1.10⁶ Mikroorganismen/cm², besonders bevorzugt zwischen etwa 5.10⁵ und etwa 1.10⁶ Mikroorganismen/cm² als praktikabel erwiesen.

Ein weiterer entscheidender Vorteil des aus einem Trägermaterial, zumindest einem Mikroorganismus und einem zumindest ein Polyacrylat enthaltenden erfindungsgemäßen biologisch aktiven Materials liegt in der Variabilität des einsetzbaren Substratmaterials.

So ermöglicht das vorstehend beschriebene Verfahren die Verwendung einer Vielzahl völlig unterschiedlich gearteter Trägermaterialien beziehungsweise unterschiedlich beschaffener Oberflächen. Das Spektrum der einsetzbaren Substrate ist sehr weit gefächert. So kommen bei Anwendungen, welche nach einer große Substratoberfläche verlangen Substrate mit poröser Oberfläche in Frage, in Anwendungen bei welchen Bekleidungsstücke mit dem biologisch aktiven Material verbunden werden, finden bevorzugt faserartige und/oder gewobene Substrate und/oder Vliese Verwendung. Für Anwendungen in welchen poröse Oberflächen aufgrund ihrer Verschmutzungsanfälligkeit und/oder aufgrund ihrer schlechten Strömungseigenschaften ungeeignet sind, ist es möglich Substrate mit porenfreier, glatter Oberfläche zu verwenden. Im Anwendungsbereich der Filtration beziehungsweise Reinigung von Flüssigkeiten sind ebenfalls aus Kunststoffschäumen und Geweben aufgebaute Substrate verwendbar.
Beispiele für die vorstehend aufgeführten Materialien sind Polyurethanschäume beziehungsweise Kunststoffschäume mit großer Oberfläche; Silicate bzw. Zeolithe, die sich durch besonders große Poren und eine große innere Oberfläche auszeichnen, was sie für die Verwendung in Katalysatorpackungen interessant macht; Beton, Fleece und gewobene Substrate wie beispielsweise Vliese und Textilien. Bei der Verwendung der vorstehend aufgeführten Substrate kann das Substrat gestanzt und/oder gefräst und/oder gewoben und/oder gepreßt sein, um eine gewünschte Form anzunehmen.

In vorteilhafter Weise schafft das erfindungsgemäße Verfahren auch die Möglichkeit, Füllkörper für Katalysatorpackungen wie beispielsweise Raschigringe, Spiralfüllkörper, zylindrische Füllkörper, gitterartige Füllkörper, Ringfüllkörper, kugelförmige Füllkörper mit einem biologisch aktiven Mikroorganismus zu verbinden. Hierdurch sind stabile und dauerhafte Katalysatorpackungen mit einer definierten Mikroorganismenbeaufschlagung herstellbar, welche je nach Ausführungsform rasch austauschbar sind und zudem geringere Druckverluste ermöglichen als bisher verwendete mit Mikroorganismen versehene Katalysatorpackungen.

Das dauerhafte Verbinden von Mikroorganismen mit Substraten unterschiedlichster Oberflächenbeschaffenheit, Struktur und darüber hinaus unterschiedlichster Stabilität stellt einen entscheidenden Vorteil gegenüber dem bisherigen Stand der Technik dar. Daher ermöglicht die vorliegende Erfindung ein für eine Anwendung spezifisches biologisch aktives Material in gewünschter Form, Porosität, Geometrie, Fläche, Größe und Stabilität herzustellen.

Als nach der Erfindung vorgeslener Bindemittel wird eine 60%ige Polymer-Dispersion eines Acrylsäureestercopolymers in Wasser, verwendet.

Die Verwendung vergleichbarer, mindestens ein Copolymer der Familie der Acrylate enthaltenden Bindemittel zur Herstellung der beanspruchten biologisch aktiven Substanz liefert vergleichbare Ergebnisse wie die vorstehend genannte Polymer-Dispersion.

Ebenso vielfältig wie die vorgehend aufgezählten Substrate ist das Spektrum der erfindungsgemäß verwendbaren Mikroorganismen zur Herstellung des biologisch aktiven Materials. So kann der Mikroorganismus eine Alge und/oder ein Bakterium und/oder eine Hefe und/oder ein Pilz und/oder eine Mischung aus diesen vorgenannten Spezies umfassen. Ebenfalls ist die Verwendung der vorstehend aufgeführten Mikroorganismen in gentechnisch veränderter Form möglich.

Die erfindungsgemäß hergestellten biologisch aktiven Materialien sind aufgrund ihrer Beständigkeit und unterschiedlichen Ausführungsformen für eine Vielzahl technischer Anwendungen geeignet. So eignen sich diese aufgrund ihrer dauerhaften Stabilität und zweckmässigen Ausführungsformen beispielsweise als Teil einer Vorrichtung zur Reinigung von Flüssigkeiten, insbesondere zur Wasseraufbereitung bzw. Wasserklärung in Aquarien und Kläranlagen. Hierbei sind Ausführungsformen besonders geeignet, bei denen die Mikroorganismen beispielsweise auf einem Substrat aus Polyurethan festgelegt sind und welche aufgrund der Ausführungsform einen möglichst geringen Strömungswiderstand bewirken.

Darüber hinaus ist die Verwendung des vorstehend beschriebenen biologischen Materials in dem Mauerwerk von Klärbecken und in der Auskleidung von Teilen der Kanalisation zum Zwecke der Abwasseraufbereitung bzw. - Klärung zum Zwecke der Vorklärung von Abwässern bestens geeignet. Mit Mikroorganismen festgelegte Bestandteile des Mauerwerks können in dieser Ausführungsform bereits in dem Kanalsystem eine Vorklärung des Abwassers beziehungsweise in Drainagen von Mülldeponien eine Entgiftung der Deponieabwässer ohne zusätzliche Einbauten oder Vorrichtungen bewirken.

Weiterhin stellen der Einsatz des biologisch aktiven Materials in Biofiltern, Biowäschern oder ähnlichen Vorrichtungen zur Zu- bzw. Abluftreinigung, beispielsweise als Bauteil einer Klimaanlage, weitere Anwendungsgebiete dar. Entscheidende Vorteile der Erfindung liegen darin, dass die dauerhaft mit dem Substrat verbundenen Mikroorganismen sich während des Reinigungsprozesses nicht von ihrem Träger ablösen und dauerhaft einheitliche Reinigungsleistungen aufweisen. Darüber hinaus stellt die Erfindung biologisch aktive Materialien zur Verfügung, welche in den vorstehend genannten Anlagen einheitliche und reproduzierbare Startbedingungen beispielsweise nach einem Filterwechsel oder bei Inbetriebnahme einer Anlage ermöglicht.

Darüber hinaus ist der Einbau der vorstehend beschriebenen Materialien in Vorrichtungen zur Dekontamination verschmutzter Flüssigkeiten von Interesse. Hier ist beispielsweise der Einsatz in einer Vorrichtung zur Beseitigung von Ölteppichen mit Hilfe von Mikroorganismen äußerst vorteilhaft. In besonderer Ausführungsform wäre in diesem Fall die Festlegung von ölabbauenden Mikroorganismen an Schwimmkörpern, welche zur Eindämmung von Ölteppichen verwendet werden, von Interesse. Dies ermöglicht eine Dekontamination bereits während der Eindämmung der kontaminierten Flüssigkeiten.
Zusätzlich ist die Verwendung der biologisch aktiven Materialien auf maritimen Off-shore Öldekontaminationseinheiten wie auf Dekontaminationsschiffen oder Ölförder- und/oder verarbeitungsstätten denkbar.

Bedingt durch seine Beständigkeit eignet sich das biologisch aktive Material ebenfalls zum Einsatz in der Reaktortechnik. So ist einerseits sein Einsatz als Bestandteil eines Bioreaktors in der Lebensmittelindustrie und andererseits eine Verwendung bei prozeßtechnischen Fermentationsverfahren in der Biotechnologie, Pharmazie und Chemie vorteilhaft möglich. Die dauerhafte Festlegung der Mikroorganismen an dem Substrat bietet bei diesen Anwendungen ein breites Spektrum an Vorteilen. So können einerseits durch das Herstellen großer Katalysatorchargen einheitliche Prozeßbedingungen, welche bei der Verwendung biologischen Materials nur schwer möglich sind, geschaffen werden. Weiterhin ermöglicht die Erfindung die Herstellung von Katalysatoren, welche in höchstem Maße beständig sind und auch unter dem Einfluß hoher Scherraten beziehungsweise Strömungsgeschwindigkeiten eingesetzt werden können. Das dauerhafte festlegen der Mikroorganismen an einem beinahe beliebig gearteten Substrat ermöglicht zusätzlich, dass nach Prozessende das biologisch aktive Material dem Fermenter beziehungsweise Bioreaktor entnommen werden kann und dessen Verwendung in einem nächsten Prozess ohne aufwendige Regenerierung und/oder Reinigung. Dass diese Vorteile aufgrund geringerer Wartungs- und Standzeiten zu deutlichen Kosteneinsparungen beitragen, liegt auf der Hand.

Besonders bevorzugt ist die Verwendung des biologisch aktiven Materials im Bereich der Medizin beziehungsweise Medizintechnkik.

So bietet die Erfindung die Möglichkeit der Herstellung von Hormonen und/oder Proteinen und/oder Pharmazeutika, insbesondere auch im Organismus von Säugetieren, oder auch in Form einer Wundauflage zur spezifischen Generierung von Wachstumsfaktoren und/oder antibiotisch wirksamen Substanzen und/oder Cytostatika. Hierdurch erschließt sich ein völlig neuartiges Feld in der Anwendung des biologisch aktiven Materials. Beispielsweise ermöglicht die Erfindung den Einsatz mit rekombinanten Mikroorrganismen versehener Implantate, welche im Organismus von Säugetieren gezielt Hormone herstellen und freisetzen. Diese Anwendung wäre unter anderem im Hinblick auf Hormonbehandlungen während der Wechseljahre, bei Schilddrüsenfehlfunktionen, bei Diabetes, zur Schwangerschaftsverhütung und Kompensation von Stoffwechselerkrankungen äußerst sinnvoll.

Weiterhin ist die Herstellung von Wundauflagen zur Versorgung von an der Wundheilung beteiligten Zellen und Gewebe mit Wachstumsfaktoren eine weitere Anwendung der Erfindung. So sind Wundauflagen, welche einerseits das Wachstum der an der Wundheilung beteiligten Zellen fördern, deren Apoptose verhindern und zusätzlich das Absterben unerwünschter in der Wunde befindlicher Mikroorganismen durch die Freisetzung von Antiobiotika bewirken, herstellbar.

Im Folgenden wird die Erfindung anhand von Ausführungsbeispielen und unter Bezugnahme auf die beigeschlossenen Zeichnungen näher erläutert.

Es zeigen:
- Figur 1:: eine schematische Darstellung eines mit einem Substrat verbundenen Mikroorganismus,
- Figur 2:: eine schematische Darstellung eines mit Mikroorganismen verbundenen Hohlzylinders, welcher sich als Bestandteil einer Katalysatorschüttung eignet,
- Figur 3:: eine schematische Darstellung eines Fermenters bzw. Rührkesselreaktors 20, in dessen Rührwerk 21, das biologisch aktive Material integriert ist,
- Figur 4:: eine schematische Darstellung eines porösen Substrats 22, welches mit Mikroorganismen verbunden ist.

Detaillierte Beschreibung bevorzugter Ausführungsformen

Bei der nachfolgenden detaillierten Beschreibung der bevorzugten Ausführungsformen sollen die Bezeichnungen Festlegen, Festlegung beziehungsweise Immobilisierung oder Verbinden von Mikroorganismen, bezeichnen daß mechanische, chemische oder andere Einflüsse auf einen Mikroorganismus so ausgeübt werden, daß dieser an einem Träger dauerhaft verweilt, beziehungsweise an diesen quasi angeheftet ist.

Im Hinblick auf die Herstellung des biologischen Materials mit den genannten immobilisierten Mikrorganismen können unterschiedliche Verfahren Verwendung finden einschließlich a) kovalentem Binden des Mikroorganismus an wasserunlösliche Träger; b) ionisches Binden des Mikroorganismus an einen wasserunlöslichen Träger; c) physikalische Adsorption des Mikroorganismus an einen Träger; d) kovalentes Quervernetzen des Mikroorganismus durch bi- oder multifunktionelle Mittel .

### Beispiele

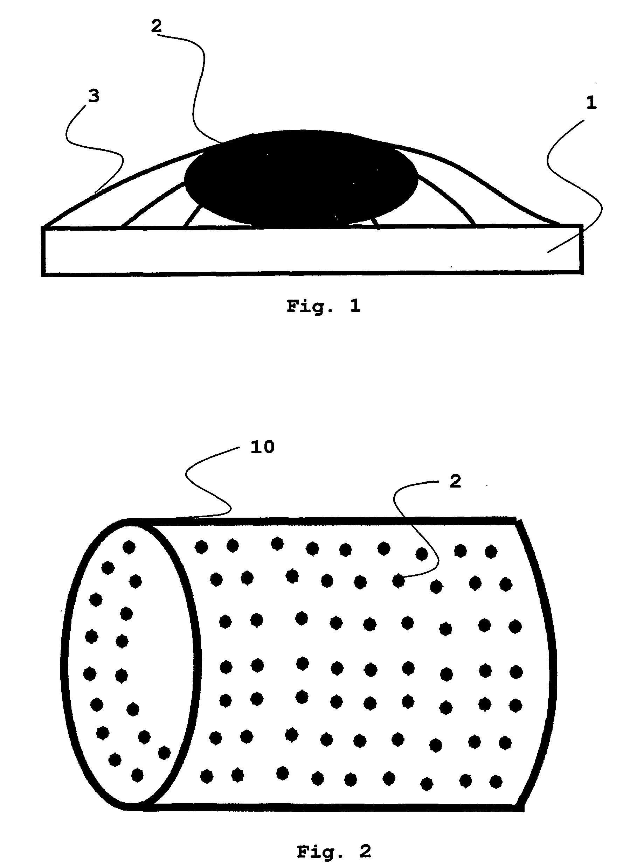

Figur 1 zeigt eine schematische Darstellung eines mit einem Substrat verbundenen Mikroorganismus.
Zu sehen ist ein Substrat 1, auf welchem ein Mikroorganismus 2 mit Hilfe eines Bindemittels 3 fixiert ist.

Wie anhand der Abbildung ersichtlich, ist der Mikroorganismus durch ein Netzwerk aus Bindemittel mit der Substratoberfläche dauerhaft verbunden. Die Versorgung der Mikroorganismen ist, bedingt durch die netzartige Struktur des Bindemittels, optimal gewährleistet.

Figur 2 zeigt eine schematische Darstellung eines mit Mikroorganismen verbundenen Hohlzylinders, welcher sich als Bestandteil einer Katalysatorschüttung eignet. Zu sehen ist ein hohlzylinderförmiges Substrat 1, welches dauerhaft mit Mikroorganismen 2 verbunden ist.

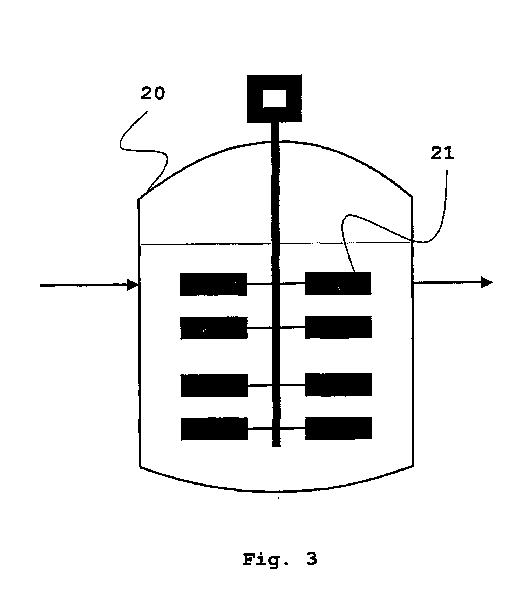

Figur 3 zeigt eine schematische Darstellung eines Fermenters bzw. Rührkesselreaktors 20, in dessen Rührwerk 21, das biologisch aktive Material integriert ist. Ein besonderer Vorteil dieser Ausführung liegt sowohl in der dauerhaften Festlegung des biologisch aktiven Materials am Rührwerk als auch darin, daß die biologisch aktive Substanz nach Beendigung der Reaktion dem Kessel problemlos entnommen und in einem weiteren Verfahren ohne aufwendige Reinigungsschritte wiederverwendet werden kann. Dies spart zum Einen Kosten und verkürzt Standzeiten, zum Andern werden einheitliche Reaktionsbedingungen ermöglicht.

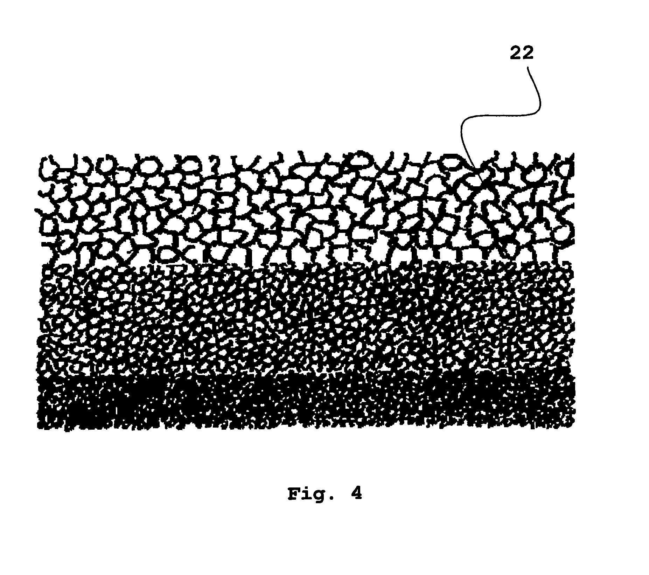

Figur 4 zeigt eine schematische Darstellung eines porösen Substrats 22, welches mit Mikroorganismen verbunden ist. Dieses poröse Substrat eignet sich besonders zur Verwendung in biologischen Filtersystemen zur Klärung und Reinigung von Flüssigkeiten.

In Figur 4 ist das Substrat in einer besonders bevorzugten Ausführungsform dargestellt. Anhand dieser Figur ist zu erkennen, daß das Substrat aus mehreren Schichten mit unterschiedlichem Vernetzungsgrad besteht. Besonders sinnvoll ist es bei dieser Verwendung die ersten Schichten zur mechanischen Vorreinigung der zu klärenden Flüssigkeit zu verwenden und die in der Abbildung unterste Schicht mit dem höchsten Vernetzungsgrad mit biologisch aktivem Material zu versehen. Bei der Verwendung solcher Filterkörper beispielsweise in Aquarienfiltern steht sofort nach dem Einbau des Filters eine optimale biologische Mikroumgebung zur Verfügung. Eine Anlaufphase bis zum Bewuchs des Filters mit Mikroorganismen, wie sie bei handelsüblichen Filtersystemen notwendig ist, entfällt.

## Patentansprüche

1. Verfahren zur Herstellung eines biologisch aktiven Materials, umfassend
- Bereitstellen eines Substrats
- Aufbringen einer Mikroorganismen enthaltenden Suspension auf das Substrat
- Verbinden des Mikroorganismus mit Hilfe eines Bindemittels, welches als 60% ige Polymer-Dispersion eines Acrylsaürestercopolymers in Wasser wobei das Pundemittel dem Mikroorganism nicht vollsteily umschließt. ausgebildet ist,

2. Verfahren zur Herstellung eines biologisch aktiven Materials nach Anspruch 1 , bei welchem die Festlegung durch das Aufbringen der Mikroorganismen an dem bereitgestellten Substrat mit Hilfe eines Bindemittels durch Ionische Wechselwirkung und/oder van der Waals-Wechselwirkung und/oder physikalische Adsorption und/oder kovalentes Quervernetzen zustande kommt.

3. Verfahren zur Herstellung eines biologisch aktiven Materials nach einem der Ansprüche von 1 bis 2, bei welchem das Bindemittel und/oder der Mikroorganismus durch Sprühen auf den Träger aufgebracht wird.

4. Verfahren zur Herstellung eines biologisch aktiven Materials nach einem der Ansprüche von 1 bis 2, bei welchem das Bindemittel und/oder der Mikroorganismus durch Tränken auf den Träger aufgebracht wird.

5. Verfahren zur Herstellung eines biologisch aktiven Materials nach den Ansprüchen 1 bis 2, **dadurch gekennzeichnet, daß** das Bindemittel und/oder der Mikroorganismus durch Schlickersuspension auf den Träger aufgebracht wird.

6. Verfahren zur Herstellung eines biologisch aktiven Materials nach einem der Ansprüche von 1 bis 2, bei welchem das Bindemittel durch Füllen von Poren auf den Träger aufgebracht wird.

7. Verfahren zur Herstellung eines biologisch aktiven Materials nach einem der Ansprüche von 1 bis 6, bei welchem das Verfahren bei einem pH Wert von etwa 4,8 bis etwa 7 durchgeführt wird.

8. Verfahren zur Herstellung eines biologisch aktiven Materials nach einem der Ansprüche von 1 bis 6, bei welchem Mikroorganismen mit einem Anteil an Bindemittel von 10 bis 90%, bevorzugt von 20 bis 60%, besonders bevorzugt von 30% vermischt werden.

9. Verfahren zur Herstellung eines biologisch aktiven Materials nach einem der Ansprüche von 1 bis 6, bei welchem Mikroorganismen mit dem Bindemittel 1 h bis 24 h, bevorzugt von 8 h bis 20 h, besonders bevorzugt von 12 h vermischt werden.

10. Verfahren zur Herstellung eines biologisch aktiven Materials nach einem der Ansprüche von 1 bis 8, bei welchem das Aufbringen der Mikroorganismen bei einer Temperatur von etwa 10 bis etwa 100 °C, bevorzugt bei 30 bis 70 °C, besonders bevorzugt bei 40 °C durchgeführt wird.

11. Verfahren zur Herstellung eines biologisch aktiven Materials nach einem der Ansprüche von 1 bis 8, bei welchem die Belegungsdichte einen Wert von etwa 1 bis 5·10⁶ Mikroorganismen/cm², bevorzugt 100.000 bis 1·10⁶ Mikroorganismen/cm², besonders bevorzugt 500.000 bis 1. 10⁶ Mikroorganismen/cm² aufweist.

12. Biologisch aktives Material, hergestellt nach einem Verfahren gemäß einem der Ansprüche von 1 bis 11, umfassend
□ ein Trägermaterial
□ zumindest einen Mikroorganismus
□ ein zumindest ein Polyacrylat enthaltendes Bindemittel.

13. Biologisch aktives Material, hergestellt nach einem Verfahren gemäß einem der Ansprüche von 1 bis 11, bei welchem das Trägermaterial eine poröse Oberfläche oder eine faserartige Oberfläche umfaßt.

14. Biologisch aktives Material, hergestellt nach einem Verfahren gemäß einem der Ansprüche von 1 bis 11, bei welchem das Trägermaterial eine Keramik umfasst.

15. Biologisch aktives Material, hergestellt nach einem Verfahren gemäß einem der Ansprüche von 1 bis 11, bei welchem das Trägermaterial einen Zeolithen umfaßt.

16. Biologisch aktives Material, hergestellt nach einem Verfahren gemäß einem der Ansprüche von 1 bis 11, bei welchem das Trägermaterial Beton umfaßt.

17. Biologisch aktives Material, hergestellt nach einem Verfahren gemäß einem der Ansprüche von 1 bis 11, bei welchem das Trägermaterial ein Vlies umfaßt.

18. Biologisch aktives Material, hergestellt nach einem Verfahren gemäß einem der Ansprüche von 1 bis 11, bei welchem das Trägermaterial ein Fleece umfaßt.

19. Biologisch aktives Material, hergestellt nach einem Verfahren gemäß einem der Ansprüche von 1 bis 11, bei welchem das Trägermaterial eine Textilie umfaßt.

20. Biologisch aktives Material, hergestellt nach einem Verfahren gemäß einem der Ansprüche von 1 bis 11, bei welchem das Trägermaterial einen Schaumstoff umfaßt.

21. Biologisch aktives Material, hergestellt nach einem Verfahren gemäß einem der Ansprüche von 1 bis 11, bei welchem das Trägermaterial Beton und einen Faserwerkstoff umfaßt.

22. Biologisch aktives Material nach den Ansprüchen 1 bis 11, bei welchem das Bindemittel ein Polycrylat umfaßt oder Copolymere der Familie der Polyacrylate enthält.

23. Biologisch aktives Material nach einem der Ansprüche von 1 bis 11, bei welchem der Mikroorganismus eine Alge und/oder ein Bakterium und/oder eine Hefe und/oder ein Pilz und/oder eine Mischung aus diesen vorgenannten Spezies umfaßt.

24. Biologisch aktives Material nach einem der Ansprüche von 1 bis 11, bei welchem der Mikroorganismus gentechnisch verändert ist.

25. Biologisch aktives Material nach einem der Ansprüche von 1 bis 11, bei welchem das Trägermaterial gestanzt, gefräst, gewoben und/oder gepreßt ist.

26. Biologisch aktives Material nach einem der Ansprüche von 1 bis 11, bei welchem der Träger ein Raschigring, Pallring, Spiralfüllkörper, zylindrischer Füllkörper, gitterartiger Füllkörper, Ringfüllkörper, eine Kugel oder ein Füllkörper für eine Katalysatorpackung ist.

27. Biologisch aktives Material nach einem der Ansprüche von 1 bis 20, bei welchem das biologisch aktive Material Teil einer Vorrichtung zur Reinigung von Flüssigkeiten, insbesondere zur Wasseraufbereitung bzw. Wasserklärung in Aquarien und/oder Kläranlagen ist.

28. Biologisch aktives Material nach einem der Ansprüche von 1 bis 26, bei welchem das biologisch aktive Material Bestandteil der Ausmauerung von Klärbecken, Kanälen und/oder Schwimmbädern ist.

29. Biologisch aktives Material nach einem der Ansprüche von 1 bis 26, bei welchem das biologisch aktive Material Teil von Gasreinigungsvorrichtungen zur Zu- bzw. Abluftreinigung in Biofiltern und/oder Biowäschern ist.

30. Biologisch aktives Material nach einem der Ansprüche von 1 bis 26, bei welchem das biologisch aktive Material Teil einer Klimaanlage ist.

31. Biologisch aktives Material nach einem der Ansprüche von 1 bis 26, bei welchem das biologisch aktive Material Teil einer Vorrichtung zur Dekontaminierung verschmutzter Flüssigkeiten ist.

32. Biologisch aktives Material nach einem der Ansprüche von 1 bis 26, bei welchem das biologisch aktive Material Teil eines Bioreaktors in der Lebensmittelindustrie ist.

33. Biologisch aktives Material nach einem der Ansprüche von 1 bis 26, bei welchem das biologisch aktive Material Teil eines Katalysators in prozeßtechnischen Fermentationsverfahren in der Biotechnologie und/oder der Chemie ist.

34. Biologisch aktives Material nach einem der Ansprüche von 1 bis 26, bei welchem das biologisch aktive Material Teil eines Katalysators in rekombinanten Herstellungsverfahren biotechnologisch produzierter Pharmazeutika ist.

35. Biologisch aktives Material nach einem der Ansprüche von 1 bis 26, bei welchem dieses Teil einer implantierbaren Ausführung zur Herstellung von Hormonen und/oder Proteinen und/oder Pharmazeutika im Organismus von Säugetieren ist.

36. Biologisch aktives Material nach einem der Ansprüche von 1 bis 26, bei welchem dieses Teil einer Wundauflage zur spezifischen Generierung von Wachstumsfaktoren und/oder antibiotisch wirksamen Substanzen und/oder Cytostatika ist.

37. Katalysator umfassend ein biologisch aktives Material, hergestellt nach einem der Ansprüche von 1 bis 11.

38. Biofilter umfassend ein biologisch aktives Material, hergestellt nach einem der Ansprüche von 1 bis 11.

39. Abwasseraufbereitungsanlage umfassend ein biologisch aktives Material, hergestellt nach einem der Ansprüche von 1 bis 11.

40. Anlage zur Klärung von Flüssigkeiten, insbesondere stationäre oder mobile Reinigungs- oder Entsorgungsanlage umfassend ein biologisch aktives Material, hergestellt nach einem der Ansprüche von 1 bis 11.

41. Klimaanlage umfassend ein biologisch aktives Material, hergestellt nach einem der Ansprüche von 1 bis 11.

42. Luftreinigungsanlage, insbesondere Zuluft- und/oder Abluftreinigungsanlage, umfassend ein biologisch aktives Material, hergestellt nach einem der Ansprüche von 1 bis 11.

43. Bioreaktor umfassend ein biologisch aktives Material, hergestellt nach einem der Ansprüche von 1 bis 11.

44. Implantierbare Vorrichtung, insbesondere human- oder veterinärmedizinisches Implantat, umfassend ein biologisch aktives Material, hergestellt nach einem der Ansprüche von 1 bis 11.

45. Wundauflage umfassend ein biologisch aktives Material, hergestellt nach einem der Ansprüche von 1 bis 11.

46. Bekleidungsstück umfassend ein biologisch aktives Material, hergestellt nach einem der Ansprüche von 1 bis 11.

47. Maritime Öldekontaminationseinheit umfassend ein biologisch aktives Material, hergestellt nach einem der Ansprüche von 1 bis 11.

## Claims

1. A method for producing a biologically active material, comprising
- providing a substrate;
- applying a microorganisms containing suspension onto the substrate;
- attaching the microorganism by means of a binder which is prepared as a 60 % polymer dispersion of an acrylic acid ester copolymer in water, wherein the microorganism is not completely enclosed by the binder.

2. The method for producing a biologically active material as claimed in claim 1, wherein attachment by applying the microorganisms to the provided substrate using a binder results from ionic interaction and/or van der Waals interaction and/or physical adsorption and/or covalent cross-linking.

3. The method for producing a biologically active material as claimed in any of claims 1 to 2, wherein the binder and/or the microorganism is applied to the substrate by spraying.

4. The method for producing a biologically active material as claimed in any of claims 1 to 2, wherein the binder and/or the microorganism is applied to the substrate by impregnating.

5. The method for producing a biologically active material as claimed in claims 1 to 2, **characterized in that** the binder and/or the microorganism is applied to the substrate by slurry suspension.

6. The method for producing a biologically active material as claimed in any of claims 1 to 2, wherein the binder is applied to the substrate by filling pores.

7. The method for producing a biologically active material as claimed in any of claims 1 to 6, the method being carried out at a pH value from about 4.8 to about 7.

8. The method for producing a biologically active material as claimed in any of claims 1 to 6, wherein microorganisms are mixed with a proportion of binder from 10 to 90 %, preferably from 20 to 60 %, more preferably of 30 %.

9. The method for producing a biologically active material as claimed in any of claims 1 to 6, wherein microorganisms are mixed with the binder for 1 h to 24 h, preferably for 8 h to 20 h, more preferably for 12 h.

10. The method for producing a biologically active material as claimed in any of claims 1 to 8, wherein applying the microorganisms is accomplished at a temperature from about 10 °C to about 100 °C, preferably from 30 °C to 70 °C, more preferably at 40 °C.

11. The method for producing a biologically active material as claimed in any of claims 1 to 8, wherein the population density is from about 1 to 5·10⁶ microorganisms/cm², preferably 100,000 to 1·10⁶ microorganisms/cm², more preferably 500,000 to 1·10⁶ microorganisms/cm².

12. A biologically active material, produced by a method as claimed in any of claims 1 to 11, comprising
- a substrate material;
- at least one microorganism;
- a binder including at least one polyacrylate.

13. A biologically active material, produced by a method as claimed in any of claims 1 to 11, wherein the substrate material comprises a porous surface or a fibrous surface.

14. A biologically active material, produced by a method as claimed in any of claims 1 to 11, wherein the substrate material comprises a ceramic.

15. A biologically active material, produced by a method as claimed in any of claims 1 to 11, wherein the substrate material comprises a zeolite.

16. A biologically active material, produced by a method as claimed in any of claims 1 to 11, wherein the substrate material comprises concrete.

17. A biologically active material, produced by a method as claimed in any of claims 1 to 11, wherein the substrate material comprises a nonwoven fabric.

18. A biologically active material, produced by a method as claimed in any of claims 1 to 11, wherein the substrate material comprises a fleece.

19. A biologically active material, produced by a method as claimed in any of claims 1 to 11, wherein the substrate material comprises a textile.

20. A biologically active material, produced by a method as claimed in any of claims 1 to 11, wherein the substrate material comprises a foam.

21. A biologically active material, produced by a method as claimed in any of claims 1 to 11, wherein the substrate material comprises concrete and a fibrous material.

22. A biologically active material as claimed in claims 1 to 11, wherein the binder comprises a polyacrylate or includes copolymers of the polyacrylate family.

23. A biologically active material as claimed in any of claims 1 to 11, wherein the microorganism comprises an alga, and/or a bacterium, and/or a yeast, and/or a fungus, and/or a combination of the aforementioned species.

24. A biologically active material as claimed in any of claims 1 to 11, wherein the microorganism is genetically modified.

25. A biologically active material as claimed in any of claims 1 to 11, wherein the substrate material is die cut, milled, woven, and/or press-molded.

26. A biologically active material as claimed in any of claims 1 to 11, wherein the substrate is a Raschig ring, a Pall ring, spiral filling material, cylindrical filling material, lattice-like filling material, annular filling material, a ball, or a filling material for a catalyst packing.

27. A biologically active material as claimed in any of claims 1 to 20, wherein the biologically active material is part of an apparatus for purifying liquids, in particular for water treatment or water purification in aquariums and/or sewage plants.

28. A biologically active material as claimed in any of claims 1 to 26, wherein the biologically active material is a component of the brick lining of sewage basins, channels, and/or swimming pools.

29. A biologically active material as claimed in any of claims 1 to 26, wherein the biologically active material is part of gas cleaning devices for cleaning supply and exhaust air in biofilters, and/or biowashers.

30. A biologically active material as claimed in any of claims 1 to 26, wherein the biologically active material is part of an air conditioning system.

31. A biologically active material as claimed in any of claims 1 to 26, wherein the biologically active material is part of a device for decontamination of polluted liquids.

32. A biologically active material as claimed in any of claims 1 to 26, wherein the biologically active material is part of a bioreactor in the food industry.

33. A biologically active material as claimed in any of claims 1 to 26, wherein the biologically active material is part of a catalyst in technological fermentation processes in biotechnology and/or chemistry.

34. A biologically active material as claimed in any of claims 1 to 26, wherein the biologically active material is part of a catalyst in recombinant production processes of biotechnologically produced pharmaceuticals.

35. A biologically active material as claimed in any of claims 1 to 26, which is part of an implantable embodiment for producing hormones and/or proteins and/or pharmaceuticals in the organism of mammals.

36. A biologically active material as claimed in any of claims 1 to 26, which is part of a wound dressing for specific generation of growth factors and/or antibiotically active substances and/or cytostatic agents.

37. A catalyst, comprising a biologically active material produced according to any of claims 1 to 11.

38. A biofilter, comprising a biologically active material produced according to any of claims 1 to 11.

39. A wastewater treatment facility, comprising a biologically active material produced according to any of claims 1 to 11.

40. A facility for purification of liquids, in particular a stationary or mobile cleaning or disposal facility, comprising a biologically active material produced according to any of claims 1 to 11.

41. An air conditioning system, comprising a biologically active material produced according to any of claims 1 to 11.

42. An air purification facility, in particular a supply air and/or exhaust air cleaning facility, comprising a biologically active material produced according to any of claims 1 to 11.

43. A bioreactor, comprising a biologically active material produced according to any of claims 1 to 11.

44. An implantable device, in particular a human or veterinary medical implant, comprising a biologically active material produced according to any of claims 1 to 11.

45. A wound dressing, comprising a biologically active material produced according to any of claims 1 to 11.

46. A garment, comprising a biologically active material produced according to any of claims 1 to 11.

47. A maritime oil decontamination unit, comprising a biologically active material produced according to any of claims 1 to 11.

## Revendications

1. Procédé pour fabriquer un matériau biologiquement actif, comprenant
- mise à disposition d'un substrat
- application d'une suspension contenant des micro-organismes sur le substrat
- liaison du micro-organisme à l'aide d'un liant, qui est conçu sous forme de dispersion de polymère diluée à 60 % d'un copolymère d'ester d'acide acrylique dans de l'eau, le liant n'entourant pas complètement le micro-organisme.

2. Procédé pour fabriquer un matériau biologiquement actif selon la revendication 1, dans lequel la fixation s'effectue par l'application des micro-organismes sur le substrat mis à disposition à l'aide d'un liant par interaction ionique et/ou interaction van der Waals et/ou adsorption physique et/ou réticulation transversale covalente.

3. Procédé pour fabriquer un matériau biologiquement actif selon l'une des revendications 1 et 2, dans lequel le liant et/ou le micro-organisme est appliqué par pulvérisation sur le support.

4. Procédé pour fabriquer un matériau biologiquement actif selon l'une des revendications 1 et 2, dans lequel le liant et/ou le micro-organisme est appliqué par imprégnation sur le support.

5. Procédé pour fabriquer un matériau biologiquement actif selon les revendications 1 et 2, **caractérisé en ce que** le liant et/ou le micro-organisme est appliqué par suspension en barbotine sur le support.

6. Procédé pour fabriquer un matériau biologiquement actif selon l'une des revendications 1 et 2, dans lequel le liant est appliqué sur le support par remplissage de pores.

7. Procédé pour fabriquer un matériau biologiquement actif selon l'une des revendications 1 à 6, dans lequel le procédé est mis en oeuvre avec un pH d'environ 4,8 jusqu'à environ 7.

8. Procédé pour fabriquer un matériau biologiquement actif selon l'une des revendications 1 à 6, dans lequel des micro-organismes sont mélangés avec une fraction de liant de 10 à 90 %, de préférence de 20 à 60 %, avec une préférence particulière de 30 %.

9. Procédé pour fabriquer un matériau biologiquement actif selon l'une des revendications 1 à 6, dans lequel des micro-organismes sont mélangés avec le liant de 1 h jusqu'à 24 h, de préférence de 8 h jusqu'à 20 h, avec une préférence particulière de 12 h.

10. Procédé pour fabriquer un matériau biologiquement actif selon l'une des revendications 1 à 8, dans lequel l'application des micro-organismes est effectuée à une température d'environ 10 jusqu'à environ 100°C, de préférence à 30 jusqu'à 70°C, avec une préférence particulière à 40°C.

11. Procédé pour fabriquer un matériau biologiquement actif selon l'une des revendications 1 à 8, dans lequel la densité d'occupation présente une valeur d'environ 1 jusqu'à 5·10⁶ micro-organismes/cm², de préférence 100 000 jusqu'à 1·10⁶ micro-organismes/cm², avec une préférence particulière pour 500 000 jusqu'à 1·10⁶ micro-organismes/cm².

12. Matériau biologiquement actif, fabriqué selon un procédé selon l'une des revendications 1 à 11, comprenant
□ un matériau support
□ au moins un micro-organisme
□ un liant contenant au moins un polyacrylate.

13. Matériau biologiquement actif, fabriqué selon un procédé selon l'une des revendications 1 à 11, dans lequel le matériau support comprend une surface poreuse ou une surface de type fibre.

14. Matériau biologiquement actif, fabriqué selon un procédé selon l'une des revendications 1 à 11, dans lequel le matériau support comprend une céramique.

15. Matériau biologiquement actif, fabriqué selon un procédé selon l'une des revendications 1 à 11, dans lequel le matériau support comprend une zéolite.

16. Matériau biologiquement actif, fabriqué selon un procédé selon l'une des revendications 1 à 11, dans lequel le matériau support comprend du béton.

17. Matériau biologiquement actif, fabriqué selon un procédé selon l'une des revendications 1 à 11, dans lequel le matériau support comprend un non-tissé.

18. Matériau biologiquement actif, fabriqué selon un procédé selon l'une des revendications 1 à 11, dans lequel le matériau support comprend un "fleece".

19. Matériau biologiquement actif, fabriqué selon un procédé selon l'une des revendications 1 à 11, dans lequel le matériau support comprend un matériau textile.

20. Matériau biologiquement actif, fabriqué selon un procédé selon l'une des revendications 1 à 11, dans lequel le matériau support comprend un produit alvéolaire.

21. Matériau biologiquement actif, fabriqué selon un procédé selon l'une des revendications 1 à 11, dans lequel le matériau support comprend du béton et un matériau fibreux.

22. Matériau biologiquement actif selon l'une des revendications 1 à 11, dans lequel le liant comprend un polyacrylate ou contient des copolymères de la famille des polyacrylates.

23. Matériau biologiquement actif selon l'une des revendications 1 à 11, dans lequel le micro-organisme comprend une algue et/ou une bactérie et/ou une levure et/ou un champignon et/ou un mélange de ces espèces susmentionnées.

24. Matériau biologiquement actif selon l'une des revendications 1 à 11, dans lequel le micro-organisme est modifié par génie génétique.

25. Matériau biologiquement actif selon l'une des revendications 1 à 11, dans lequel le matériau support est découpé, fraisé, tissé et/ou comprimé.

26. Matériau biologiquement actif selon l'une des revendications 1 à 11, dans lequel le support est une bague Raschig, une bague Pall, un corps de remplissage en spirale, un corps de remplissage cylindrique, un corps de remplissage de type grille, un corps de remplissage en bague, une boule ou un corps de remplissage pour un emballage catalyseur.

27. Matériau biologiquement actif selon l'une des revendications 1 à 20, ledit matériau biologiquement actif faisant partie d'un dispositif pour nettoyer des liquides, en particulier pour le traitement de l'eau ou l'épuration de l'eau dans des aquariums et/ou des stations d'épuration.

28. Matériau biologiquement actif selon l'une des revendications 1 à 26, ledit matériau biologiquement actif faisant partie de la maçonnerie de bassins de clarification, de canaux et/ou de piscines.

29. Matériau biologiquement actif selon l'une des revendications 1 à 26, ledit matériau biologiquement actif faisant partie de dispositifs de nettoyage de gaz pour le nettoyage de l'air arrivant ou de l'air sortant dans des biofiltres et/ou des biolaveurs.

30. Matériau biologiquement actif selon l'une des revendications 1 à 26, ledit matériau biologiquement actif faisant partie d'une installation de climatisation.

31. Matériau biologiquement actif selon l'une des revendications 1 à 26, ledit matériau biologiquement actif faisant partie d'un dispositif pour la décontamination de liquides encrassés.

32. Matériau biologiquement actif selon l'une des revendications 1 à 26, ledit matériau biologiquement actif fait partie d'un bioréacteur dans l'industrie alimentaire.

33. Matériau biologiquement actif selon l'une des revendications 1 à 26, ledit matériau biologiquement actif faisant partie d'un catalyseur dans des procédés de fermentation technologiques en biotechnologie et/ou en chimie.

34. Matériau biologiquement actif selon l'une des revendications 1 à 26, ledit matériau biologiquement actif faisant partie d'un catalyseur dans des procédés de fabrication recombinants de produits pharmaceutiques produits par biotechnologie.

35. Matériau biologiquement actif selon l'une des revendications 1 à 26, celui-ci faisant partie d'une réalisation implantable pour la fabrication d'hormones et/ou de protéines et/ou de produits pharmaceutiques dans l'organisme de mammifères.

36. Matériau biologiquement actif selon l'une des revendications 1 à 26, celui-ci faisant partie d'un support de plaie pour la génération spécifique de facteurs de croissance et/ou de substances actives au plan antibiotique et/ou de cytostatiques.

37. Catalyseur comprenant un matériau biologiquement actif, fabriqué selon l'une des revendications 1 à 11.

38. Biofiltre comprenant un matériau biologiquement actif, fabriqué selon l'une des revendications 1 à 11.

39. Installation de traitement d'eaux usées comprenant un matériau biologiquement actif, fabriqué selon l'une des revendications 1 à 11.

40. Installation pour clarifier des liquides, en particulier installation de nettoyage ou d'évacuation fixe ou mobile comprenant un matériau biologiquement actif, fabriqué selon l'une des revendications 1 à 11.

41. Installation de climatisation comprenant un matériau biologiquement actif, fabriqué selon l'une des revendications 1 à 11.

42. Installation d'épuration d'air, en particulier installation d'épuration d'air arrivant et/ou d'air sortant, comprenant un matériau biologiquement actif, fabriqué selon l'une des revendications 1 à 11.

43. Bioréacteur comprenant un matériau biologiquement actif, fabriqué selon l'une des revendications 1 à 11.

44. Dispositif implantable, en particulier implant de médecine humaine ou de médecine vétérinaire, comprenant un matériau biologiquement actif, fabriqué selon l'une des revendications 1 à 11.

45. Support de plaie comprenant un matériau biologiquement actif, fabriqué selon l'une des revendications 1 à 11.

46. Vêtement comprenant un matériau biologiquement actif, fabriqué selon l'une des revendications 1 à 11.

47. Unité maritime de décontamination de mazout, comprenant un matériau biologiquement actif, fabriqué selon l'une des revendications 1 à 11.
